# EUROPEAN PATENT SPECIFICATION

(11) **EP 4 281 143 B1**
(45) Date of publication and mention of the grant of the patent: **13.05.2026**
(21) Application number: 21848480.6
(22) Date of filing: 30.12.2021
(51) Int. Cl.: A61M 5/142, A61M 5/148

(54) **OCCLUSION SENSOR FOR PUMP ASSEMBLY**
OKKLUSIONSSENSOR FÜR PUMPENANORDNUNG
CAPTEUR D'OCCLUSION POUR ENSEMBLE POMPE

(30) Priority: 20.01.2021 US 202163139664 P
(43) Date of publication of application: 29.11.2023
(73) Proprietor: Becton, Dickinson and Company, Franklin Lakes, NJ 07417-1880 (US)
(72) Inventor: TUOHY, John, Ashford, Co. Wicklow, A67YK70 (IE)
(74) Representative: dompatent
(86) International application number: PCT/US2021/065699
(87) International publication number: WO 2022/159242

(56) References cited:
- WO-A2-2017/053284
- US-A1- 2013 283 934
- US-B2- 10 668 212

## Description

### Field of the Invention

The present invention relates to an occlusion sensor in a system comprising a pump assembly to detect an occlusion in the system.

### Background of the Invention

Diabetes is a group of diseases characterized by high levels of blood glucose resulting from the inability of diabetic patients to maintain proper levels of insulin production when required. Diabetes can be dangerous to the affected patient if it is not treated, and it can lead to serious health complications and premature death. However, such complications can be minimized by utilizing one or more treatment options to help control the diabetes and reduce the risk of complications.

The treatment options for diabetic patients include specialized diets, oral medications and/or insulin therapy. The main goal of diabetes treatment is to control the diabetic patient's blood glucose or sugar level. However, maintaining proper diabetes management may be complicated because it has to be balanced with the activities of the diabetic patient. Type 1 diabetes (T1D) patients are required to take insulin (e.g., via injections or infusion) to move glucose from the bloodstream because their bodies generally cannot produce insulin. Type 2 diabetes (T2D) patients generally can produce insulin but their bodies cannot use the insulin properly to maintain blood glucose levels within medically acceptable ranges. In contrast to people with T1D, the majority of those with T2D usually do not require daily doses of insulin to survive. Many people are able to manage their condition through a healthy diet and increased physical activity or oral medication. However, if they are unable to regulate their blood glucose levels, they will be prescribed insulin. For example, there are an estimated 6.2 million Type 2 diabetes patients (e.g., in the United States, Western Europe and Canada) taking multiple-daily-injections (MDI) which consist of a 24-hour basal insulin and a short acting rapid insulin that is taken at mealtimes for glycemic management control.

For the treatment of Type 1 diabetes (T1D) and sometimes Type 2 diabetes (T2D), there are two principal methods of daily insulin therapy. In the first method, diabetic patients use syringes or insulin pens to self-inject insulin when needed. This method requires a needle stick for each injection, and the diabetic patient may require three to four injections daily. The syringes and insulin pens that are used to inject insulin are relatively simple to use and cost effective.

Another effective method for insulin therapy and managing diabetes is infusion therapy or infusion pump therapy in which an insulin pump is used. The insulin pump can provide continuous infusion of insulin to a diabetic patient at varying rates to more closely match the functions and behavior of a properly operating pancreas of a non-diabetic person that produces the required insulin, and the insulin pump can help the diabetic patient maintain his/her blood glucose level within target ranges based on the diabetic patient's individual needs. Infusion pump therapy requires an infusion cannula, typically in the form of an infusion needle or a flexible catheter, that pierces the diabetic patient's skin and through which infusion of insulin takes place. Infusion pump therapy offers the advantages of continuous infusion of insulin, precision dosing, and programmable delivery schedules.

In infusion therapy, insulin doses are typically administered at a basal rate and in a bolus dose. When insulin is administered at a basal rate, insulin is delivered continuously over 24 hours to maintain the diabetic patient's blood glucose levels in a consistent range between meals and rest, typically at nighttime. Insulin pumps may also be capable of programming the basal rate of insulin to vary according to the different times of the day and night. In contrast, a bolus dose is typically administered when a diabetic patient consumes a meal, and generally provides a single additional insulin injection to balance the consumed carbohydrates. Insulin pumps may be configured to enable the diabetic patient to program the volume of the bolus dose in accordance with the size or type of the meal that is consumed by the diabetic patient. In addition, insulin pumps may also be configured to enable the diabetic patient to infuse a correctional or supplemental bolus dose of insulin to compensate for a low blood glucose level at the time when the diabetic patient is calculating the bolus dose for a particular meal that is to be consumed.

Insulin pumps advantageously deliver insulin over time rather than in single injections, typically resulting in less variation within the blood glucose range that is recommended. In addition, insulin pumps may reduce the number of needle sticks which the diabetic patient must endure, and improve diabetes management to enhance the diabetic patient's quality of life. For example, many of the T2D patients who are prescribed insulin therapy can be expected to convert from injections to infusion therapy due to an unmet clinical need for improved control. That is, a significant number of the T2D patients who take multiple-daily-injections (MDI) are not achieving target glucose control or not adhering sufficiently to their prescribed insulin therapy.

Typically, regardless of whether a diabetic patient uses multiple direct injections (MDIs) or a pump, the diabetic patient takes fasting blood glucose medication (FBGM) upon awakening from sleep, and also tests for glucose in the blood during or after each meal to determine whether a correction dose is required. In addition, the diabetic patient may test for glucose in the blood prior to sleeping to determine whether a correction dose is required, for instance, after eating a snack before sleeping.

To facilitate infusion therapy, there are generally two types of insulin pumps, namely, conventional pumps and patch pumps. Conventional pumps use a disposable component, typically referred to as an infusion set, tubing set or pump set, which conveys the insulin from a reservoir within the pump into the skin of the user. The infusion set includes a pump connector, a length of tubing, and a hub or base from which a cannula, in the form of a hollow metal infusion needle or flexible plastic catheter, extends. The base typically has an adhesive that retains the base on the skin surface during use. The cannula can be inserted onto the skin manually or with the aid of a manual or automatic insertion device. The insertion device may be a separate unit employed by the user.

Another type of insulin pump is a patch pump. Unlike a conventional infusion pump and infusion set combination, a patch pump is an integrated device that combines most or all of the fluidic components in a single housing. Generally, the housing is adhesively attached to an infusion site on the patient's skin, and does not require the use of a separate infusion or tubing set. A patch pump containing insulin adheres to the skin and delivers the insulin over a period of time via an integrated subcutaneous cannula. Some patch pumps may wirelessly communicate with a separate controller device (as in one device sold by Insulet Corporation under the brand name OmniPod^{®}), while others are completely self-contained. Such patch pumps are replaced on a frequent basis, such as every three days, or when the insulin reservoir is exhausted. Otherwise, complications may occur, such as restriction in the cannula or the infusion site.

As a patch pump is designed to be a self-contained unit that is worn by the patient, preferably, the patch pump is small, so that it does not interfere with the activities of the user. In current patch pump designs, tubes, such as plastic tubes, are employed as fluid pathways to route fluid flow from one internal component to another. For example, a tube can connect a medicament reservoir with a delivery needle. Typically, medicament is drawn from the medicament reservoir via a vacuum. However, because of the enclosed nature of the patch pump, it can be difficult to determine if an occlusion is present. Further, noise, fluid contact and other factors can influence and make it difficult to identify the presence of an occlusion. Accordingly, a need exists for an improved system and method to determine an occlusion in the system prior to or during medicament delivery.

US10668212B2 discloses an invention related to medical pumping devices and more precisely to the detection of dysfunctions in such devices, such as occlusions.

WO2017053284A2 discloses a filling member in a medicament delivery device , the filling member includes a first conduit that fluidly communicates with a reservoir and a second conduit that fluidly communicates with a pump and with the first conduit, wherein the filling member provides two-way medicament flow that enters the reservoir via the first conduit, exits the reservoir into the first conduit and the second conduit, and exits the second conduit to the pump. The reservoir includes a reservoir tube having one end that is formed with the reservoir, and the reservoir tube having another end that is press fit to the filling member to establish fluid communication with the reservoir including an occlusion sensor.

US20130283934A1 discloses an apparatus provided for sensor assemblies and related medical devices. An embodiment of a sensor assembly includes a rigid structure and a beam structure having an outer portion in contact with the rigid structure and an inner portion. The beam structure includes one or more beams extending between the outer portion and the inner portion of the beam structure and a cantilevered portion extending from the inner portion to inhibit displacement of the inner portion toward the rigid structure. Each beam has a sensing element disposed thereon.

### Summary of Embodiments of the Invention

It is an aspect of the present invention to provide occlusion detection in a system such a patch pump. A strain gauge is bonded onto a mid-section of an outer surface of a flexible reservoir to provide sensitive strain reading. The bonding surfaces are strong and repeatable adhering surfaces regardless of the medicament consistency and rate of medicament flow exiting the flexible reservoir. Accordingly, the occlusion is advantageously detected directly from the flexible reservoir and not at a location downstream as the medicament travels to the cannula for medication delivery. The occlusion detection is advantageously not dependent on contacting the medicament or other noise factors commonly experienced using other types of occlusion sensors. Thus, the strain gauge in the patch pump provides a simple, cost effective and effective means to determine if an occlusion is present.

The foregoing and/or other aspects of the present invention can be achieved by providing a medicament delivery system comprising a reservoir that carries a medicament, a pump connected to the reservoir that draws the medicament out of the reservoir for medication delivery, and an occlusion sensor connected to the reservoir that senses whether an occlusion is present.

The foregoing and/or other aspects of the present invention can be further achieved by providing a method for determining an occlusion in a medicament delivery system, the method comprising securing an occlusion sensor to an outer surface of a flexible reservoir, operating the medicament delivery system for medication delivery, measuring resistance at the outer surface of the flexible reservoir, providing resistance output from the occlusion sensor to control electronics, and determining whether an occlusion is present based on the resistance output.

The foregoing and/or other aspects of the present invention can be also achieved by providing a medicament delivery system wherein the reservoir comprises a flexible reservoir.

The foregoing and/or other aspects of the present invention can additionally be achieved by providing a medicament delivery system wherein the occlusion sensor comprises a strain gauge, the occlusion sensor does not contact the medicament, the occlusion sensor does not detect pressure, the occlusion sensor does not detect current draw, and the occlusion sensor outputs resistance to control electronics.

The foregoing and/or other aspects of the present invention can further be achieved by providing a medicament delivery system wherein the resistance decays as the medicament exits the reservoir when there is no occlusion, the occlusion is detected when the resistance does not decay according to a designated pattern and as the medicament exits the reservoir, a surface of the reservoir compresses, and the occlusion sensor senses changes in forces across the reservoir.

The foregoing and/or other aspects of the present invention can also be achieved by providing a medicament delivery system wherein as the medicament exits the reservoir, tension at a surface of the reservoir reduces and the tension is measured axially across the reservoir.

The foregoing and/or other aspects of the present invention can additionally be achieved by providing a medicament delivery system wherein the occlusion sensor is insensitive to lateral forces across the reservoir, the occlusion prevents the medicament from exiting the reservoir, and the occlusion sensor is bonded to a mid-section of an outer surface of the reservoir.

Finally, the foregoing and/or other aspects of the present invention can also be achieved by providing a method for securing the occlusion sensor to a mid-section of the outer surface of the flexible reservoir, measuring axial tension and axial compression at the outer surface of the flexible reservoir to determine resistance and determining whether the occlusion is present based on a decay of the resistance output.

Additional and/or other aspects and advantages of the present invention will be set forth in the description that follows, or will be apparent from the description, or may be learned by practice of the invention. The present invention may comprise delivery devices and methods for forming and operating the same having one or more of the above aspects, and/or one or more of the features and combinations thereof. The present invention may comprise one or more of the features and/or combinations of the above aspects as recited, for example, in the attached claims.

### Brief Description of the Drawings

The above and/or other aspects and advantages of embodiments of the invention will be more readily appreciated from the following detailed description, taken in conjunction with the accompanying drawings, of which:
Fig. 1 is a perspective view of a fluidic architecture and metering sub-system diagram of an exemplary patch pump;
Fig. 2 illustrates a wireless remote controller for controlling the operation of a medication delivery device such as, for example, a patch pump, in accordance with an illustrative embodiment of the present invention;
Fig. 3 is a perspective view of a patch pump in accordance with an illustrative embodiment of the present invention;
Fig. 4 is a cross-sectional view taken along line 7-7 of Fig. 3;
Fig. 5 is a perspective view of a reservoir tube connected to the reservoir in a patch pump in accordance with an illustrative embodiment of the present invention;
Fig. 6 is perspective view of the reservoir connected to the filling member in a patch pump in accordance with an illustrative embodiment of the present invention;
Fig. 7 is a perspective view of the reservoir and a receptacle used to connect to the filling member, in a patch pump in accordance with an illustrative embodiment of the present invention;
Fig. 8 is a perspective view of the reservoir of Fig. 7 without the receptacle in a patch pump in accordance with an illustrative embodiment of the present invention;
Fig. 9 is a top perspective view of a strain gauge in accordance with an illustrative embodiment of the present invention;
Fig. 10 is a top perspective view of a patch pump configured to engage the strain gauge of Fig. 9;
Fig. 11 is a right side simplified cross-sectional view of a patch pump configured to engage the strain gauge of Fig. 9;
Fig. 12 is a top view of a flexible reservoir in a patch pump configured to engage the strain gauge of Fig. 9; and
Fig. 13 is a schematic describing the operation of the strain gauge of Fig. 9.

### Detailed Description of Embodiments of the Present Invention

Reference will now be made in detail to embodiments of the present invention, which are illustrated in the accompanying drawings, wherein like reference numerals refer to like elements throughout. The embodiments described herein exemplify, but do not limit, the present invention by referring to the drawings.

It will be understood by one skilled in the art that this disclosure is not limited in its application to the details of construction and the arrangement of components set forth in the following description or illustrated in the drawings. The inventions described herein are capable of other embodiments, and capable of being practiced or carried out in various ways. Also, it will be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not restricted to physical or mechanical connections or couplings. Further, terms such as up, down, bottom, and top are relative, and are employed to aid illustration, but are not limiting.

The illustrative embodiments are described with reference to diabetes management using insulin therapy. However, it is to be understood that these illustrative embodiments can be used with different drug therapies and regimens to treat other physiological conditions other than diabetes using medicaments other than insulin.

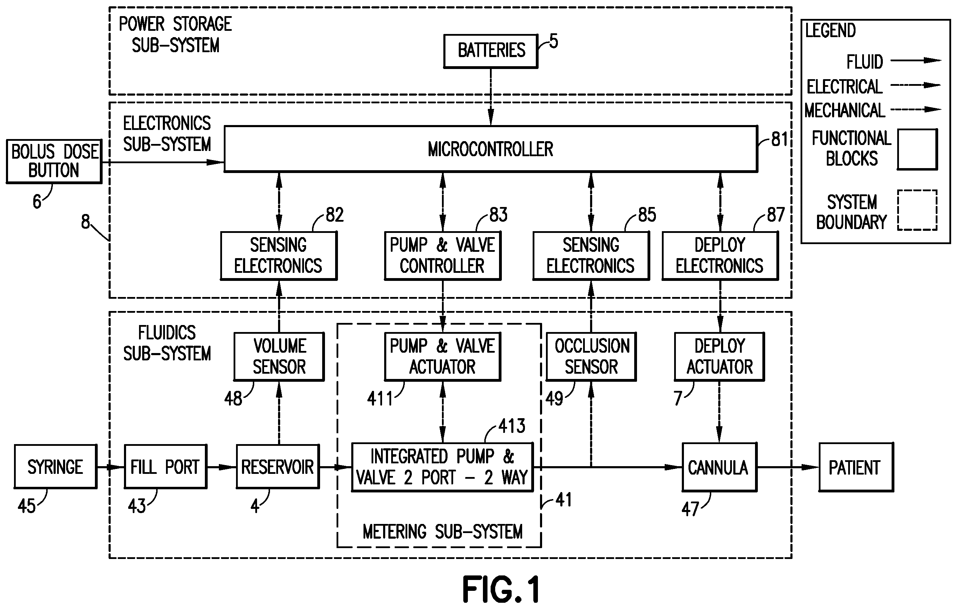

Figs. 1 through 4 depict an exemplary embodiment of a medicament delivery device comprising a patch pump 1. The various components of the patch pump 1 may include: a reservoir 4 for storing insulin or other liquid medicament; a pump 3 for pumping insulin out of the reservoir 4; a power source 5 in the form of one or more batteries; an insertion mechanism 7 for inserting an inserter needle with a catheter into a user's skin; control electronics 8 in the form of a circuit board with optional communications capabilities to outside devices such as a remote controller and computer, including a smart phone; a pair of dose buttons 6 on the cover 2 for actuating an insulin dose, including a basal and/or bolus dose; and a base 9 to which various components above may be attached via fasteners. The patch pump 1 also includes various fluid connector lines that transfer insulin pumped out of the reservoir 4 to the infusion site.

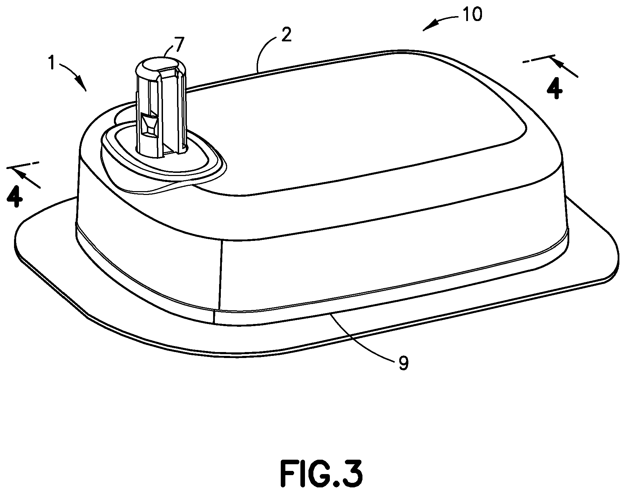

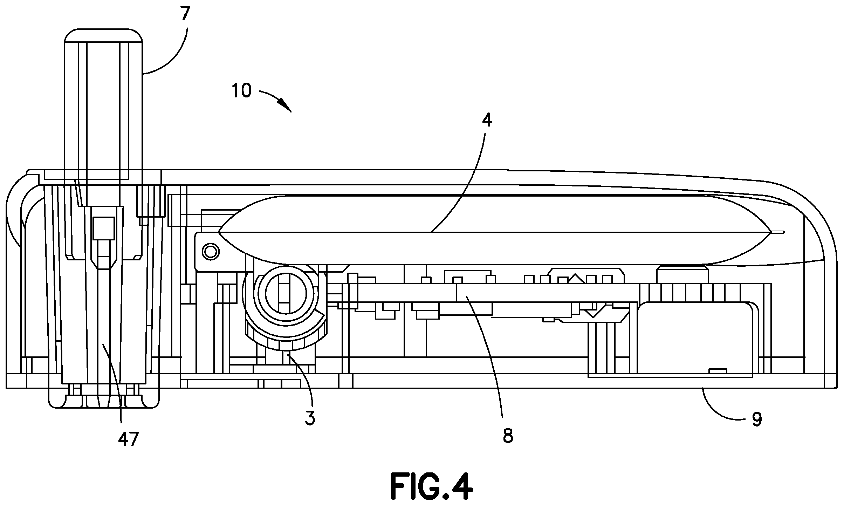

With reference to Figs. 3 and 4, a patch pump 1 can have a flexible reservoir 4 which can reduce the external dimensions of the patch pump 1 since the flexible reservoir 4 can fill voids within the patch pump 1. The patch pump 1 is illustrated with a cannula insertion device 7 that inserts the cannula, typically at an acute angle, less than 90 degrees, at the surface of a user's skin. The patch pump 1 further comprises: a power source 5 in the form of batteries; a metering sub-system 41 that monitors the volume of insulin and includes a low volume detecting ability; control electronics 8 for controlling the components of the device; and a reservoir fill port 43 for receiving a refill syringe to fill the reservoir 4.

Fig. 1 is a fluidic architecture and metering sub-system diagram for the patch pump 1. The power storage sub-system for the patch pump 1 includes the batteries 5. The control electronics 8 of the patch pump 1 may include a microcontroller 81, sensing electronics 82, pump and valve controller 83, sensing electronics 85, and deployment electronics 87, which control the actuation of the patch pump 1. The patch pump 1 includes a fluidics sub-system that may include a reservoir 4, volume sensor 48 for the reservoir 4, a reservoir fill port 43 for receiving a refill syringe 45 to refill the reservoir 4. The fluidics sub-system may include a metering system comprising a pump and valve actuator 411 and an integrated pump and valve mechanism 413. The fluidics sub-system may further include an occlusion sensor 49, a deploy actuator 7, as well as the cannula 47 for insertion into an infusion site on the user's skin.

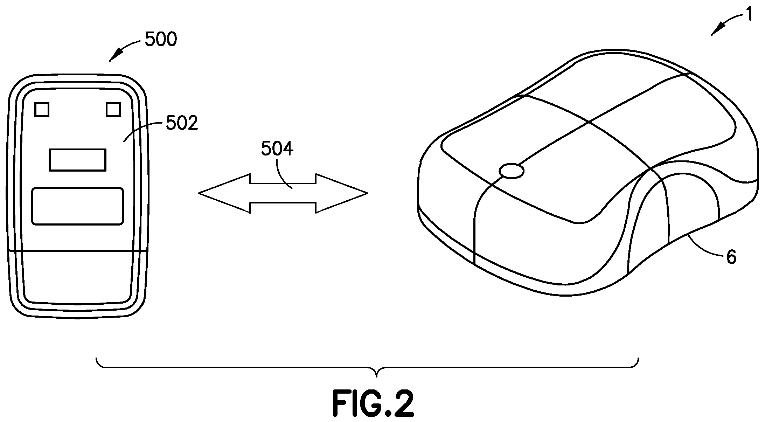

With reference to Fig. 2, the wearable medical delivery device (e.g., insulin delivery device (IDD) such as patch pump 1) is optionally operable in conjunction with a remote controller that preferably communicates wirelessly with the pump 1 and is hereinafter referred to as the wireless controller (WC) 500. The WC can comprise a graphical user interface (GUI) display 502 for providing a user visual information about the operation of the patch pump 1 such as, for example, configuration settings, an indication when a wireless connection to the patch pump is successful, and a visual indication when a dose is being delivered, among other display operations. The GUI display 502 can include a touchscreen display that is programmed to allow a user to provide touch inputs such as a swipe to unlock, swipe to confirm a request to deliver a bolus, and selection of confirmation or settings buttons, among other user interface operations.

The WC 500 can communicate with the delivery device (e.g., patch pump 1) using any one or more of a number of communication interfaces 504. For example, a near field radiation interface is provided to synchronize the timing of the WC and patch pump 1 to facilitate pairing upon start up. Another interface can be provided for wireless communication between the WC and the patch pump 1 that employs a standard BlueTooth Low Energy (BLE) layer, as well as Transport and Application layers. Non-limiting examples of Application layer commands include priming, delivering basal dose, delivering bolus dose, cancelling insulin delivery, checking patch pump 1 status, deactivating the patch pump , and patch pump 1 status or information reply.

Fig. 3 is a perspective view of a patch pump 1 according to an exemplary embodiment of the present invention. The patch pump 1 has a housing 10, which includes a main cover 2 liquid sealed or, preferably, hermetically sealed to a base 9. The base 9 carries various components as described below in detail. The hermetic seal prevents fluid ingress and prevents other particles from passing the seal. Embodiments of the patch pump 1 also include a vent or a vent membrane along with a sealing method described herein to provide pressure equalization.

Embodiments of the seal include, for example, a liquid-tight seal, an O-ring seal or another mechanical seal, a gasket, an elastomer, a heat seal, an ultra-sonically welded seal, a laser weld, chemical joining, an adhesive, a solvent weld, or an adhesive weld. Laser welding is the preferred sealing method because when laser welding is properly performed, a seamless fully hermetic seal is formed. The vent or the vent membrane continues to have the functional purpose of equalizing internal pressure and providing a sterile environment. One skilled in the art will appreciate that other seals can be used without departing from the scope of the present invention.

Fig. 4 is a cross-sectional view of the patch pump 1 illustrating various internal components. The main cover 2 and the base 9 house the components of the patch pump 1. According to one embodiment, the patch pump 1 preferably includes a reservoir 4 for storing medicament (such as insulin) and a pump 3 for pumping the medicament to exit the reservoir 4. The patch pump 1 also preferably includes electronics 8 for programming and operating the patch pump 1, and an insertion mechanism 7 for inserting a cannula 47 into a skin of the patient to deliver medicament. Examples of the electronics 8 include semiconductor chips, controllers, diodes, antennas, coils, batteries, discrete components (resistors and capacitors, for example) and circuit boards used to operate and control the patch pump 1 and operate the pump 1 in conjunction with the WC 500.

The reservoir 4 can either be a flexible reservoir or a rigid reservoir. Typically, a device having a rigid reservoir does not use a pump. Rather, a piston operates inside the rigid reservoir to drive the medicament out of the reservoir, into the flow path and through the various components of the device, and administer the medicament to the patient. On the other hand, a device having a flexible reservoir typically uses a pump within the device. The medicament is pulled from the reservoir by the pump, pushed through the various components of the device, and administered to the patient. Preferably, the patch pump 1 incorporates a flexible reservoir design where the reservoir 4 does not include a piston. Instead, the medicament is pulled from the reservoir 4 by the pump 3, and the pump 3 is external to the reservoir 4.

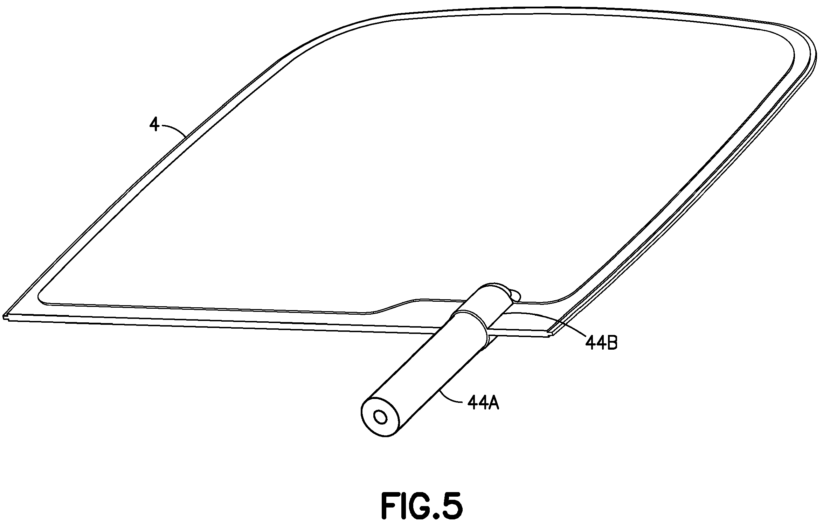

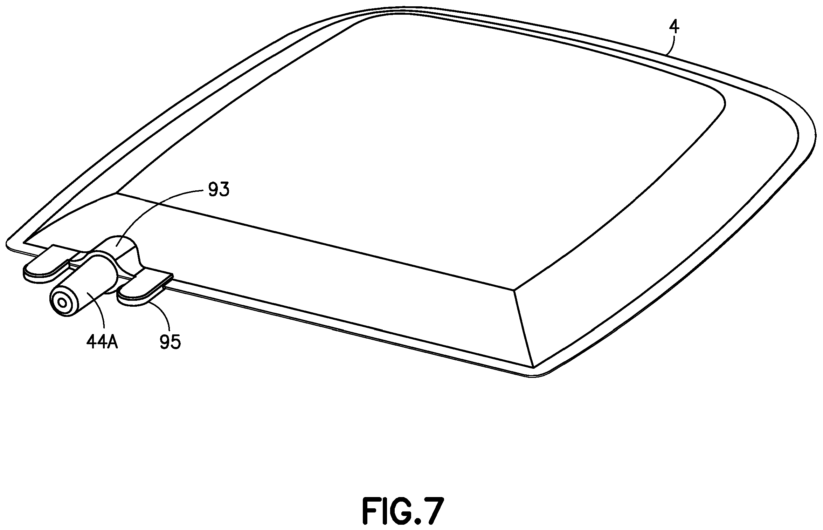

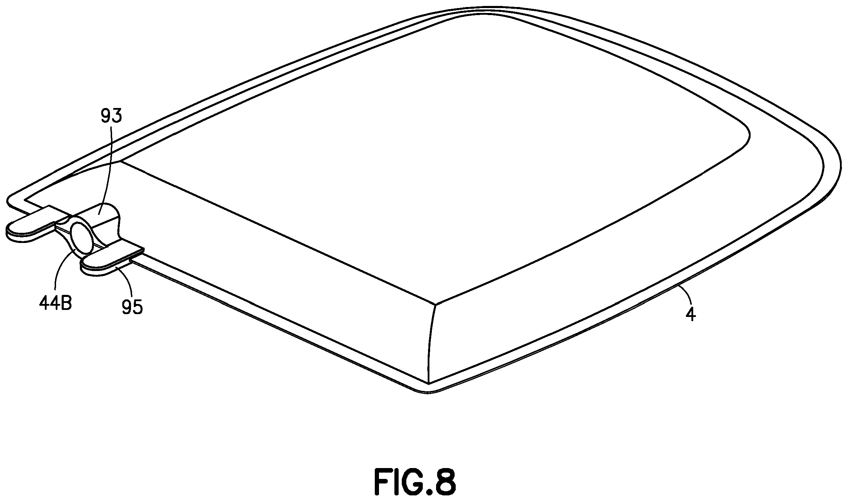

Figs. 5-8 depict alternative illustrative embodiments for a reservoir port connector or joint 44B that connects a reservoir tube 44A to the reservoir 4. The reservoir 4 is of a compact, smaller size compared to what is generally used in the industry. The reservoir 4 comprises a flexible, collapsible reservoir made from film materials ranging in thickness between 0,00508 - 0.0381 cm (.002- .015 inches). The thickness can be varied depending on the need for structural integrity, flexibility, barrier properties, filling/emptying operational behavior and drug type. For example, material-type and thickness can be selected to accommodate a selected pressure (e.g., which is affected by how much fluid is being delivered and by fluid properties), to preserve the integrity of reservoir 4 during shipping and handling, to achieve desired flexibility to conform to the reservoir port 44B or to a tube 44A and to prevent leakage of reservoir fluid, and/or to achieve a desired fill rate and/or volume.

Barrier properties include non-blocking characteristics that are considered in film material selection so that the film does not stick to itself as it collapses during emptying and blocks insulin flow. Barrier characteristic selection prevents contamination of the contents of the reservoir 4 (e.g., by external gases such as room air or fluids such as condensation). The material of the reservoir 4 can consist of one or more layers. For example, a three layer material can be used with an internal layer with properties conducive to heat sealing to a tube 44A and one or more outer layers having the afore-mentioned barrier properties or characteristics to prevent contamination of the contents of the reservoir 4 and protection of the integrity of the reservoir 4 during shipping, handling and use.

The film perimeter is sealed according to a variety of methods such as heat-sealing, radio frequency welding, laser welding, or other joining techniques that cause melting of the two film faces together. The preferred material of the reservoir 4 is sealed Air M312A film that is heat sealed. This material is advantageously compatible to insulin over an extended period of time up to at least three days. Additionally, the reservoir 4 is packaged with an oil film to protect the reservoir 4 during storage and prior to operation.

The reservoir 4 can be formed in a variety of ways. According to one embodiment, the reservoir 4 is formed by using two film sheets at each of the top and bottom surfaces that flexibly goes around the reservoir tube 44A. Such a configuration can provide optimal sealing between the reservoir 4 and the reservoir tube 44A. According to another embodiment, the reservoir 4 is formed by folding a single film on one edge and sealing the remaining edges. In another embodiment, the reservoir 4 may be formed by taking a tubular film and sealing at two opposite ends. The reservoir 4 is formed in another embodiment by using a rigid backing on the top surface and a flexible film on the bottom surface. During the perimeter sealing process, the reservoir 4 can be formed in any desired shape. The reservoir 4 can also be formed to include features to enable attachment to specific anchor points in the patch pump 1 for mounting purposes. The reservoir 4 satisfies industry sterilization and aging requirements and all operational loads/conditions.

Figs. 5-8 also illustrate a receptacle 93 in more detail. In particular, the receptacle 93 can include the flexible reservoir tube 44A as a single unitary structure, or as a separate tube that is press fit or otherwise secured to a recess of the receptacle 93. Two flanges 95 are disposed on either side of the receptacle 93 to increase the surface area and thus strengthen the bond between the receptacle 93 and the reservoir 4 as described above. Additionally, the two flanges 95 improve assembly of the reservoir 4 because the flanges 95 provide a surface for a user to hold the receptacle 93.

In operation, the reservoir 4 is prefilled in a device or filled in the patch pump 1 prior to use by providing an appropriate filling port. When the flexible reservoir 4 is filled, it will expand to a final, filled shaped that is dependent on material properties, size and shape. When the reservoir 4 is connected to the pump 3 during operation, the fluid is driven and withdrawn from the reservoir 4. The reservoir 4 generally immediately collapses (self-collapsing) by an amount equal to the volume of fluid removed. The flexibility of the film of the reservoir 4 allows for the emptying (reservoir collapsing) behavior. The flexibility of the reservoir 4 advantageously provides optimal use of the internal volume of the patch pump 1. The fluid subsequently travels to the filling member 43 upon exiting the reservoir 4 and the receptacle 93.

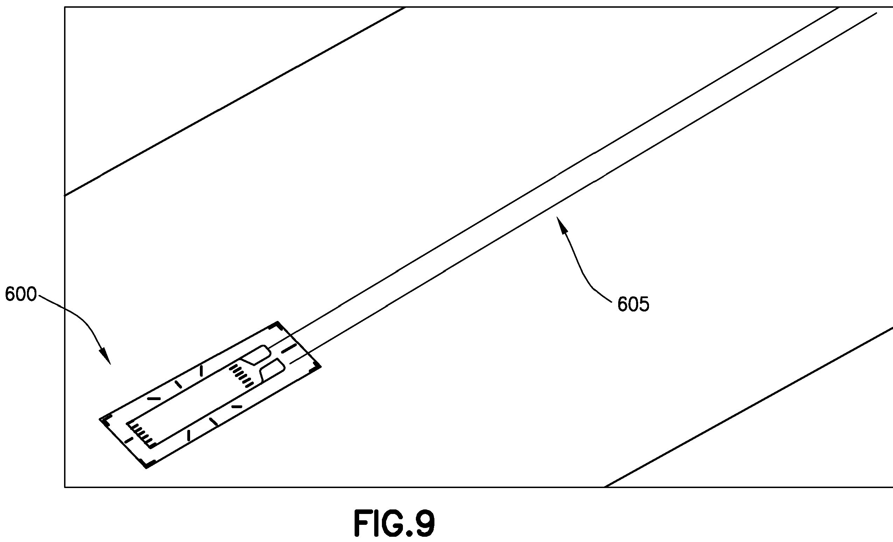

Figs. 9-13 illustrate an occlusion sensor 600 configured to be secured to a patch pump 1 according to an exemplary embodiment of the present invention. An occlusion in a drug delivery system, such as the patch pump 1, could potentially mean that a pump 3 is active but not distributing fluid from the reservoir 4. Thus, it is important to detect an occlusion quickly and accurately to prevent inaccuracies in dosing.

The occlusion sensor 600 preferably comprises a strain gauge. The occlusion sensor 600 is connected to the reservoir 4, and senses whether an occlusion is present. The occlusion sensor 600 also includes wiring 605 that electrically connects to the control electronics 8 of the patch pump 1. Specifically, the control electronics 8 provide electrical current for the occlusion sensor 600 to operate. Resistance output received by the occlusion sensor 600 during operation of the patch pump 1 is then communicated to the control electronics 8 for processing and occlusion determination. The occlusion sensor 600 does not detect current draw.

Any of the flexible reservoirs 4 carrying the medicament as described above can be used in this embodiment. A flexible reservoir 4 that is pressurized and sealed with medicament is advantageously selected to cooperate with the strain gauge 600. A rigid reservoir 4 would not function properly with the strain gauge 600 for reasons described below.

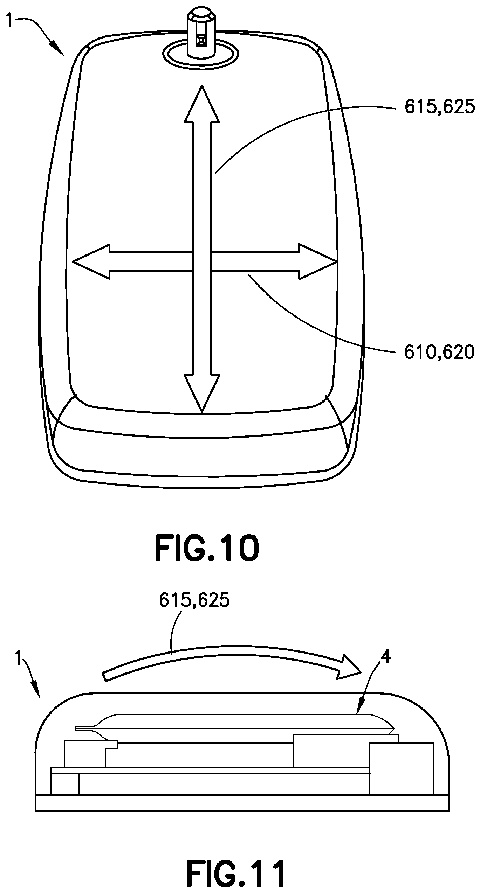

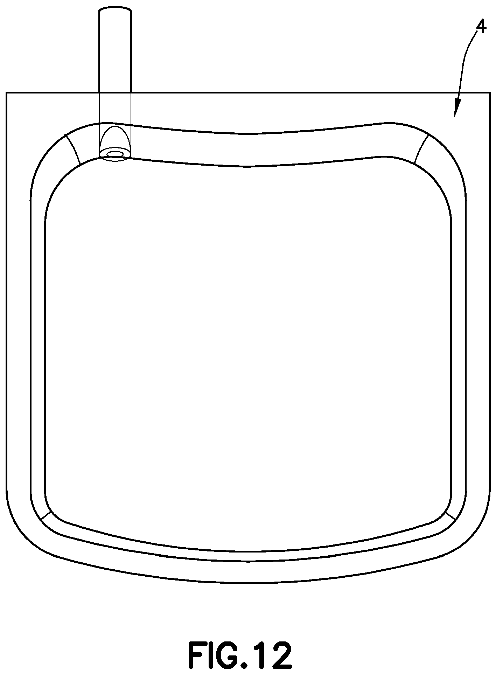

Figs. 10-12 illustrate the flexible reservoir 4 in a filled state (Figs. 10 and 11) and an empty state (Fig. 12). The pump 3, as described in the embodiments above, is connected to the flexible reservoir 4 and draws the medicament out of the flexible reservoir 4 to the cannula 47 for medication delivery.

The strain gauge 600 includes any of a variety of gauges as understood by one skilled in the art such as an RS PRO Wire Lead Strain Gauge 3.5mm, 120Ω -30°C +80°C, and an I.E.E. Strain Gauge 27.8mm, >1MΩ -30°C +170°C. The strain gauge 600 is bonded onto an outer surface of the flexible reservoir 4 to measure occlusion in the patch pump 1. The strain gauge 600 can be bonded to the flexible reservoir 4 either before calibration and filling of the flexible reservoir 4 or after filling of the flexible reservoir 4 but before medication delivery. The bonding material of the adhesive and application thereof is conventionally understood by one skilled in the art.

Attachment of the strain gauge 600 is challenging because a volume of medicament in the flexible reservoir 4 changes during calibration and operation, as well as the resulting shape of the flexible reservoir 4. Specifically, during calibration, the flexible reservoir 4 is filled with the medicament and inflates like a balloon. Figs. 10 and 11 show the curved flexible reservoir 4 with a relatively flat mid-section. When filled, the flexible reservoir 4 in an on body injector (OBI) like the patch pump 1 can have an output pressure of approximately 20psi. Subsequently, during operation, the medicament slowly exits the flexible reservoir 4 causing the flexible reservoir 4 to naturally deflate.

For several advantageous reasons, the strain gauge 600 is bonded to an outer surface of the mid-section of the flexible reservoir 4 where its flattest portion is located. This position provides the strongest strain when the flexible reservoir 4 is filled, provides a strong and repeatable adhering surface, and thus provides the most sensitive measurement reading for the strain gauge 600.

The strain gauge 600 ideally should be at the mid-section or central to the flexible reservoir 4 on its top surface. This configuration advantageously avoids the pump 3 disposed near a bottom inner surface of the base 9. Also, the mid-section or center of the flexible reservoir 4 endures the greatest strain and therefore provides the greatest signal for the strain gauge 600 to read.

If the strain gauge 600 is bonded at one of the ends of the flexible reservoir 4, the strain gauge 600 will have difficulty obtaining accurate strain measurements. For example, if the strain gauge 600 is positioned at an end of the flexible reservoir 4 opposite to the receptacle 93, the compression of that portion of the flexible reservoir 4 during medicament delivery will be less pronounced and thus more difficult to measure, especially at the beginning and end of medicament delivery. If the strain gauge 600 is positioned near the receptacle 93, that portion of the flexible reservoir 4 may not experience much compression until near the end of medicament delivery.

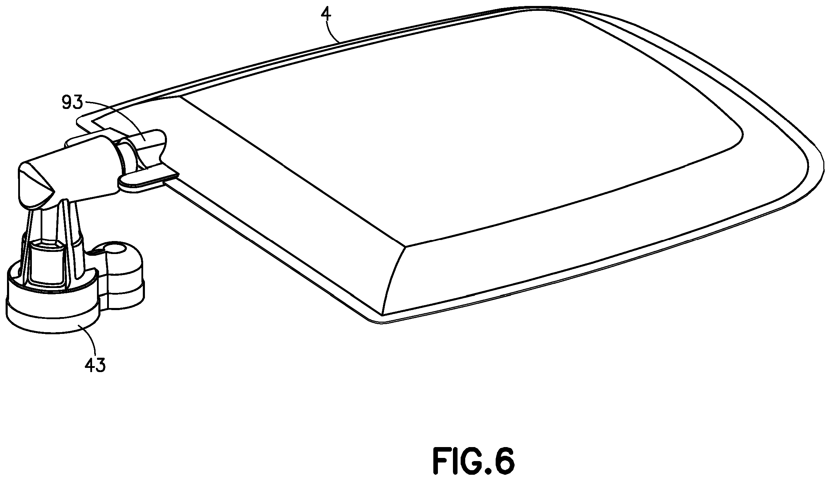

Further, maintaining attachment of the strain gauge 600 to the flexible reservoir 4 will be the strongest at the mid-section of the flexible reservoir 4 where it is the flattest (e.g., in the middle portion of the reservoir body versus its respective end portions or sides). As a result, in mass production, adhering the strain gauge 600 to the mid-section of the flexible reservoir 4 will provide consistent attachment and optimal reliability. As illustrated in Figs. 6-8, sides of the flexible reservoir 4 are not flat and its shape will change significantly during the course of medication delivery. Accordingly, it will be difficult to maintain a consistent and reliable attachment between the strain gauge 600 and the flexible reservoir 4 outside the mid-section of the flexible reservoir 4. On the other hand, the mid-section or central portion of the flexible reservoir 4 may not change as significantly because its flat nature will largely remain the same.

Furthermore, attaching the strain gauge 600 to the mid-section of the flexible reservoir 4 also advantageously avoids issues related to inconsistent or uneven fluid flow and/or a variable fluid flow rate of the medicament out of the flexible reservoir 4. This is because the flexible reservoir 4 is pressurized and sealed with medicament after calibration. Any form of medicament flow exiting the flexible reservoir 4 regardless of the consistency or the flow rate will cause the flexible reservoir 4 to deflate and create a change in resistance for the strain gauge 600 to measure and output to the control electronics 8 of the patch pump 1.

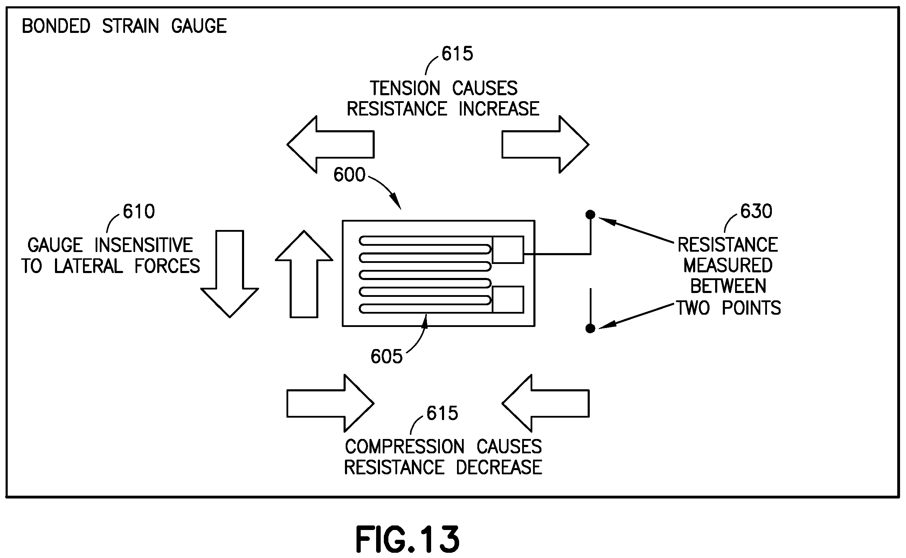

Fig. 13 shows that the strain gauge 600 measuring the tension and/or compression 615 in the flexible reservoir 4 to determine if medicament is exiting the flexible reservoir 4 during operation of the patch pump 1. Figs. 6-8, 10 and 11 illustrate that the tension/compression 615 is measured axially between two points on an axial plane 625 parallel to a plane connecting the receptacle 93 to an opposing surface of the flexible reservoir 4. A lateral plane 620 of the flexible reservoir 4 is a plane perpendicular to the axial plane 625 as described above. Specifically, the lateral plane 620 includes a plane connecting opposing surfaces of the flexible reservoir 4 but does not include a plane parallel to the plane connecting the receptacle 93 to an opposing surface of the flexible reservoir 4.

Specifically, as illustrated in Fig. 13, when the flexible reservoir 4 is filled with medicament and inflates like a balloon, a maximum amount of tension 615 is experienced at an outer surface of a middle portion of the flexible reservoir 4. In this configuration, the strain gauge 600 measures a high resistance or tension 630. As the medicament exits the flexible reservoir 4, the outer surface of the middle portion of the flexible reservoir 4 compresses or reduces in tension 615. As a result, the resistance 630 measured by the strain gauge 600 decays or decreases in a parabolic manner or other designated pattern. The decay rate is dependent on various factors including but not limited to the size and shape of the flexible reservoir 4, the form factor of the flexible reservoir 4, the flexibility of the material of the flexible reservoir 4, and the flow rate of the medicament. The resistance 630 is outputted by the strain gauge 600 to the control electronics 8 of the patch pump 1 via the wiring 605.

During operation of the patch pump 1, if the resistance 630 of the strain gauge 600 is not decaying or decreasing according to an expected rate, the control electronics 8 determines that there is an occlusion present that is preventing the medicament from exiting the flexible reservoir 4. The user is subsequently alerted. The strain gauge 600 is advantageously insensitive to lateral forces 610 experienced in the lateral plane 620 as described above. Movement of the medicament in the lateral plane 620 of the flexible reservoir 4 may be misleading and not correspond to fluid exiting the flexible reservoir 4. This is because medicament movement in the lateral plane 620 is perpendicular to a flow direction required to exit the receptacle 93 of the flexible reservoir 4.

The strain gauge 600 is an alternate, more convenient and cost effective occlusion sensor than a typical pressure sensor commonly understood by one skilled in the art. As described above, the strain gauge 600 uniquely operates only on flexible reservoirs 4 and receives feedback directly from the flexible reservoir 4. Thus, the occlusion is advantageously detected directly from the flexible reservoir 4 and not from a location downstream as the medicament travels to the cannula 47 for medication delivery. Also, the detection of the occlusion is advantageously not dependent on other noise factors such as current draw or integrated pressure sensors in the fluid path which contact the medicament, for example.

A conventional occlusion sensor is typically identified by a visual indicator based on restriction of an actuating wire movement of a plunger, for example, or a pressure sensor that measures pressure of the medicament in a fluid line of the patch pump 1. Specifically, most patch pumps 1 have a rigid reservoir including a solid cylinder that incorporates a piston to push out the medicament. If the piston stops moving, an occlusion is obviously present. The challenge that a flexible reservoir 4 provides is that occlusions are not visually obvious. Thus, the strain decay of the flexible reservoir 4 can be a beneficial indicator of a successful medicament delivery.

The strain gauge 600 according to this embodiment does not measure pressure but rather advantageously measures strain of the flexible reservoir 4 as described above to provide a simple, cost effective, yet accurate means to determine if an occlusion is present. Moreover, the strain gauge 600 advantageously does not contact the medicament. Thus, the strain gauged 600 is easy to install and incorporate into the patch pump 1.

Although only a few embodiments of the present invention have been shown and described, the present invention is not limited to the described embodiments. Instead, it will be appreciated by those skilled in the art that changes may be made to these embodiments without departing from the principles of the invention. It is particularly noted that those skilled in the art can readily combine the various technical aspects and elements of the various exemplary embodiments that have been described above in numerous other ways, all of which are considered to be within the scope of the invention, which is defined by the appended claims.

## Claims

1. A medicament delivery system comprising:
a reservoir (4) that carries a medicament;
a pump (3) connected to the reservoir (4) that draws the medicament out of the reservoir (4) for medication delivery; and an occlusion sensor (49) connected to the reservoir (4) that senses whether an occlusion is present,
the reservoir (4) comprising a flexible reservoir,
the occlusion sensor (49) comprising a strain gauge,
wherein
the occlusion sensor (49) does not contact the medicament.

2. The system according to claim **1,** wherein the occlusion sensor (49) does not detect pressure.

3. The system according to claim **1,** wherein the occlusion sensor (49) does not detect current draw.

4. The system according to claim **1,** wherein the occlusion sensor (49) outputs resistance to control electronics (8).

5. The system according to claim 4, wherein the resistance decays as the medicament exits the reservoir (4) when there is no occlusion.

6. The system according to claim 4, wherein the occlusion (49) is detected when the resistance of the strain gauge (600) does not decay according to a designated pattern.

7. The system according to claim **1,** wherein as the medicament exits the reservoir (4), a surface of the reservoir (4) compresses, and the occlusion sensor (49) senses changes in forces across the reservoir (4).

8. The system according to claim **1,** wherein as the medicament exits the reservoir (4), tension at a surface of the reservoir (4) reduces.

9. The system according to claim 8, wherein the tension is measured axially across the reservoir (4).

10. The system according to claim **1,** wherein the occlusion sensor (49) is insensitive to lateral forces across the reservoir (4).

11. The system according to claim **1,** wherein the occlusion sensor (49) is bonded to a mid-section of an outer surface of the reservoir (4).

12. A method for determining an occlusion (49) in a medicament delivery system, the method comprising:
securing an occlusion sensor (49) to an outer surface of a flexible reservoir (4);
operating the medicament delivery system for medication delivery;
measuring resistance at the outer surface of the flexible reservoir (4);
providing resistance output from the occlusion sensor (49) to control electronics (8); and
determining whether an occlusion (49) is present based on the resistance output,
wherein the reservoir (4) comprises a flexible reservoir,
wherein the occlusion sensor (49) comprises a strain gauge,
wherein
the occlusion sensor (49) does not contact the medicament.

13. The method of claim 12, further comprising securing the occlusion sensor (49) to a mid-section of the outer surface of the flexible reservoir (4).

14. The method of claim 12, further comprising measuring axial tension and axial compression at the outer surface of the flexible reservoir (4) to determine resistance.

15. The method of claim 12, further comprising determining whether the occlusion (49) is present based on a decay of the resistance output.

## Patentansprüche

1. Medikamentenabgabesystem, das aufweist:
ein Reservoir (4), das ein Medikament enthält;
eine Pumpe (3), die mit dem Reservoir (4) verbunden ist und das Medikament zur Medikamentenabgabe aus dem Reservoir (4) ansaugt; und einen Okklusionssensor (49), der mit dem Reservoir (4) verbunden ist und feststellt, ob eine Okklusion vorhanden ist,
wobei das Reservoir (4) ein flexibles Reservoir aufweist,
wobei der Okklusionssensor (49) einen Dehnungsmessstreifen aufweist,
wobei
der Okklusionssensor (49) nicht mit dem Medikament in Kontakt kommt.

2. System nach Anspruch 1, wobei der Okklusionssensor (49) keinen Druck erfasst.

3. System nach Anspruch 1, wobei der Okklusionssensor (49) keine Stromaufnahme erfasst.

4. System nach Anspruch 1, wobei der Okklusionssensor (49) einen Widerstand an die Steuerelektronik (8) ausgibt.

5. System nach Anspruch 4, wobei der Widerstand abfällt, wenn das Medikament aus dem Reservoir (4) austritt, wenn keine Okklusion vorliegt.

6. System nach Anspruch 4, wobei die Okklusion (49) erkannt wird, wenn der Widerstand des Dehnungsmessstreifens (600) nicht gemäß einem bestimmten Muster abfällt.

7. System nach Anspruch 1, wobei beim Austritt des Medikaments aus dem Reservoir (4) eine Oberfläche des Reservoirs (4) komprimiert wird und der Okklusionssensor (49) Änderungen der Kräfte über das Reservoir (4) erfasst.

8. System nach Anspruch 1, wobei beim Austritt des Medikaments aus dem Reservoir (4) die Spannung an der Oberfläche des Reservoirs (4) abnimmt.

9. System nach Anspruch 8, wobei die Spannung axial über das Reservoir (4) gemessen wird.

10. System nach Anspruch 1, wobei der Okklusionssensor (49) unempfindlich gegenüber seitlichen Kräften über das Reservoir (4) ist.

11. System nach Anspruch 1, wobei der Okklusionssensor (49) mit einem mittleren Abschnitt einer Außenfläche des Reservoirs (4) verbondet ist.

12. Verfahren zum Bestimmen einer Okklusion (49) in einem Medikamentenabgabesystem, wobei das Verfahren umfasst:
Befestigen eines Okklusionssensors (49) an einer Außenfläche eines flexiblen Reservoirs (4);
Betätigen des Medikamentenabgabesystems zur Medikamentenabgabe;
Messen des Widerstands an der Außenfläche des flexiblen Reservoirs (4);
Bereitstellen eines Widerstandsausgangs vom Okklusionssensor (49) für die Steuerelektronik (8); und
Bestimmen, ob eine Okklusion (49) vorhanden ist, basierend auf der Widerstandsausgabe,
wobei das Reservoir (4) ein flexibles Reservoir aufweist,
wobei der Okklusionssensor (49) einen Dehnungsmessstreifen aufweist,
wobei
der Okklusionssensor (49) nicht mit dem Medikament in Kontakt kommt.

13. Verfahren nach Anspruch 12, das ferner das Befestigen des Okklusionssensors (49) an einem mittleren Abschnitt der Außenfläche des flexiblen Reservoirs (4) umfasst.

14. Verfahren nach Anspruch 12, das ferner das Messen der axialen Spannung und der axialen Kompression an der Außenfläche des flexiblen Reservoirs (4) umfasst, um den Widerstand zu bestimmen.

15. Verfahren nach Anspruch 12, das ferner das Bestimmen, ob die Okklusion (49) vorhanden ist, basierend auf einem Abfall der Widerstandsausgabe umfasst.

## Revendications

1. Système d'administration de médicament comprenant :
un réservoir (4) qui contient un médicament ;
une pompe (3) reliée au réservoir (4) qui aspire le médicament hors du réservoir (4) pour l'administration de médicament ; et un capteur d'occlusion (49) relié au réservoir (4) qui détecte si une occlusion est présente,
le réservoir (4) comprenant un réservoir flexible,
le capteur d'occlusion (49) comprenant une jauge de contrainte,
dans lequel
le capteur d'occlusion (49) n'est pas en contact avec le médicament.

2. Système selon la revendication 1, dans lequel le capteur d'occlusion (49) ne détecte pas la pression.

3. Système selon la revendication 1, dans lequel le capteur d'occlusion (49) ne détecte pas la consommation de courant.

4. Système selon la revendication 1, dans lequel le capteur d'occlusion (49) fournit une résistance à une électronique de commande (8).

5. Système selon la revendication 4, dans lequel la résistance diminue à mesure que le médicament sort du réservoir (4) lorsqu'il n'y a pas d'occlusion.

6. Système selon la revendication 4, dans lequel l'occlusion (49) est détectée lorsque la résistance de la jauge de contrainte (600) ne diminue pas selon un motif désigné.

7. Système selon la revendication 1, dans lequel, à mesure que le médicament sort du réservoir (4), une surface du réservoir (4) se comprime et le capteur d'occlusion (49) détecte les changements de forces à travers le réservoir (4).

8. Système selon la revendication 1, dans lequel, à mesure que le médicament sort du réservoir (4), une tension à une surface du réservoir (4) se réduit.

9. Système selon la revendication 8, dans lequel la tension est mesurée axialement à travers le réservoir (4).

10. Système selon la revendication 1, dans lequel le capteur d'occlusion (49) est insensible à des forces latérales à travers le réservoir (4).

11. Système selon la revendication 1, dans lequel le capteur d'occlusion (49) est fixé à une section médiane d'une surface externe du réservoir (4).

12. Procédé pour déterminer une occlusion (49) dans un système d'administration de médicament, le procédé comprenant :
la fixation d'un capteur d'occlusion (49) à une surface externe d'un réservoir flexible (4) ;
le fonctionnement du système d'administration de médicament pour l'administration de médicament ;
la mesure de la résistance à la surface externe du réservoir flexible (4) ;
la fourniture d'une sortie de résistance du capteur d'occlusion (49) à une électronique de commande (8) ; et
la détermination si une occlusion (49) est présente sur la base de la sortie de résistance,
dans lequel le réservoir (4) comprend un réservoir flexible,
dans lequel le capteur d'occlusion (49) comprend une jauge de contrainte,
dans lequel le capteur d'occlusion (49) n'est pas en contact avec le médicament.

13. Procédé selon la revendication 12, comprenant en outre la fixation du capteur d'occlusion (49) à une section médiane de la surface externe du réservoir flexible (4).

14. Procédé selon la revendication 12, comprenant en outre la mesure d'une tension axiale et d'une compression axiale à la surface externe du réservoir flexible (4) pour déterminer la résistance.

15. Procédé selon la revendication 12, comprenant en outre la détermination si l'occlusion (49) est présente sur la base d'une diminution de la sortie de résistance.
